Europäisches Patentamt

European Patent Office

Office européen des brevets

(19)

(11) Veröffentlichungsnummer : **0 125 567**
**B1**

(12) **EUROPÄISCHE PATENTSCHRIFT**

(45) Veröffentlichungstag der Patentschrift :
22.01.86

(21) Anmeldenummer : 84104990.1

(22) Anmeldetag : 03.05.84

(51) Int. Cl.⁴ : **C 07 C 69/67**, **C 07 C 67/333**,
**C 07 C 67/38**

(54) **Verfahren zur Herstellung von delta-Formylvaleriansäureestern.**

(30) Priorität : 11.05.83 DE 3317164

(43) Veröffentlichungstag der Anmeldung :
21.11.84 Patentblatt 84/47

(45) Bekanntmachung des Hinweises auf die Patenterteilung : 22.01.86 Patentblatt 86/04

(84) Benannte Vertragsstaaten :
BE CH DE FR GB IT LI NL

(56) Entgegenhaltungen :
EP-A- 0 031 100
DE-A- 2 643 205
US-A- 3 253 018

(73) Patentinhaber : BASF Aktiengesellschaft
Carl-Bosch-Strasse 38
D-6700 Ludwigshafen (DE)

(72) Erfinder : Schneider, Heinz-Walter, Dr.
Bruesseler Ring 43
D-6700 Ludwigshafen (DE)
Erfinder : Kummer, Rudolf, Dr.
Kreuzstrasse 6
D-6710 Frankenthal (DE)
Erfinder : Taglieber, Volker, Dr.
Franz-Liszt-Strasse 15
D-6904 Eppelheim (DE)

**Beschreibung**

Wie aus Bull. Chem. Soc. of Japan am Band 46, Seite 528 und der US-PS 3 253 018 bekannt ist, erhält man δ-Formylvaleriansäureester durch Hydroformylierung von Pentensäureestern in Gegenwart von Kobaltcarbonyl-Katalysatoren. Hierbei entstehen jedoch erhebliche Mengen an verzweigten Estern, die für die weitere Verwendung unerwünscht sind. Es wurde auch versucht, den in dem Pentenestergemisch vornehmlich enthaltenen 3-Pentensäureester zu 4-Pentensäureester zu isomerisieren. Entsprechend Bull. Chem. Soc. of Japan, Band 46, Seite 528 entsteht jedoch bei der Isomerisierung von 3-Pentensäureestern mit Kobaltcarbonyl vornehmlich 2-Pentensäureester.

Bei der Isomerisierung des 3-Pentensäureesters liegen nach Erreichen des thermodynamischen Gleichgewichts 5 Isomere, nämlich 4-Pentensäureester, cis- und trans-3-Pentensäureester sowie cis- und trans 2-Pentensäureester vor, wobei das Gleichgewicht stark nach der Seite des trans-2-Pentensäureesters verschoben ist.

Es war deshalb die technische Aufgabe gestellt, die Hydroformylierung von Pentensäureestern so zu gestalten, daß möglichst wenig verzweigte Verbindungen entstehen und 3-Pentensäureester so zu isomerisieren, daß im wesentlichen eine lineare Verschiebung der Doppelbindung zu 4-Pentensäureester eintritt unter gleichzeitiger Minimierung des Anfalls an schwer abtrennbaren cis-2-Pentensäureester.

Diese Aufgabe wird gelöst in einem Verfahren zur Herstellung von δ-Formylvaleriansäureestern durch Umsetzen von Pentensäureestern mit Kohlenmonoxid und Wasserstoff unter erhöhtem Druck und bei erhöhter Temperatur in Gegenwart von Carbonylkomplexen der 8. Gruppe des periodischen Systems, dadurch gekennzeichnet, daß man

a) 3-Pentensäureester bei erhöhter Temperatur mit Isomerisierungs-Katalysatoren behandelt und 4-Pentensäureester aus dem Reaktionsgemisch abdestilliert und

b) den so erhaltenen 4-Pentensäureester mit Kohlenmonoxid und Wasserstoff bei einer Temperatur von 80 bis 160 °C und unter einem Druck von 1 bis 30 bar in Gegenwart von Rhodium-Carbonyl-Komplexen, die mit tertiären organischen Phosphinen oder -Phosphiten modifiziert sind, umsetzt.

Das neue Verfahren hat den Vorteil, daß bei der Isomerisierung von 3-Pentensäureestern wenig schwer abtrennbare cis-2-Pentensäureester gebildet werden und die Katalysatoren eine lange Lebensdauer haben. Ferner hat das neue Verfahren den Vorteil, daß es gelingt, δ-Formylvaleriansäureester herzustellen, wobei verzweigte Verbindungen nur in untergeordneten Mengen anfallen.

Das neue Verfahren ist insofern bemerkenswert, als die Verwendung von Rhodium-Katalysatoren für die Herstellung von δ-Formylvaleriansäureester durch Hydroformylierung von Pentensäureestern ausweislich der US-PS 3 253 018 als ungeeignet beschrieben wird, da diese Katalysatoren zur Bildung von im wesentlichen verzweigten Aldehyden führen.

Bevorzugt geht man von 3-Pentensäureestern, die sich von Alkoholen mit bis zu 12 Kohlenstoffatomen herleiten, aus.

Besonders bevorzugt werden 3-Pentensäurealkylester, insbesondere solche von Alkoholen mit 1 bis 4 Kohlenstoffatomen. Geeignete 3-Pentensäureester sind beispielsweise 3-Pentensäuremethylester, 3-Pentensäureethylester, 3-Pentensäurebutylester.

Die 3-Pentensäureester werden mit Isomerisierungs-Katalysatoren behandelt, z. B. mit sauren Ionenaustauschern, sauren Zeolithen, Cobaltcarbonyl oder modifizierten Rhodiumtriphenylphosphin-Komplexen.

Bevorzugt verwendet man stark saure Ionenaustauscher, z. B. vernetzte Polystyrole mit sauren Gruppen, insbesondere Sulfonsäuregruppen. Besonders bewährt haben sich Styrol-divinylbenzol-copolymerisate mit Sulfonsäuregruppen. Bevorzugte Zeolithe sind A, X oder Y-Zeolithe in ihrer H-Form, d. h. in saurer Form. Die sauren Ionenaustauscher oder sauren Zeolithe haben einen Gehalt an Edelmetallen der 8. Gruppe des periodischen Systems, insbesondere an Palladium, Rhodium oder Ruthenium. Von den genannten Metallen wird Palladium besonders bevorzugt. Vorteilhaft beträgt der Metallgehalt 0,01 bis 1 Gew.-%.

Die Isomerisierung wird bei erhöhter Temperatur durchgeführt. Vorteilhaft hält man eine Temperatur von 80 bis 180 °C ein. Falls man saure Ionenaustauscher verwendet, haben sich Temperaturen von 80 bis 140 °C bewährt. Vorteilhaft führt man die Isomerisierung bei einer Temperatur durch, die dem Siedepunkt des jeweils entstehenden 4-Pentensäureesters unter dem angewandten Druck entspricht. In der Regel führt man die Isomerisierung unter Atmosphären-Druck durch. Es ist jedoch auch möglich, verminderten oder schwach erhöhten Druck z. B. bis zu 2 bar anzuwenden.

Vorteilhaft hält man eine Verweilzeit von 0,05 bis 1 Stunde ein. Aus dem Reaktionsgemisch wird 4-Pentensäureester abdestilliert und das verbleibende Gemisch zweckmäßig wiederum der Isomerisierung zugeführt.

Der so erhaltene 4-Pentensäureester wird mit Kohlenmonoxid und Wasserstoff umgesetzt. In der Regel enthält das Gasgemisch Kohlenmonoxid und Wasserstoff im Molverhältnis 4 : 1 bis 1 : 4. Vorteilhaft wendet man ein Gemisch aus Kohlenmonoxid und Wasserstoff an, das die Bestandteile in etwa äquimolaren Mengen enthält. Bezogen auf 4-Pentensäureester wendet man das Gemisch aus Kohlenmonoxid und Wasserstoff in der Regel von stöchiometrischen Mengen bis zu einem Überschuß von 20 % an.

Die Hydroformylierung wird bei einer Temperatur von 80 bis 140 °C durchgeführt. Vorteilhaft wendet man eine Temperatur von 90 bis 120 °C an. Ferner führt man die Umsetzung unter einem Druck von 1 bis 30 bar durch. Vorteilhaft hält man einen Druck von 8 bis 20 bar ein.

Die Umsetzung wird in Gegenwart von Rhodium-Carbonyl-Komplexen, die mit tertiären organischen Phosphinen oder -phosphiten modifiziert sind, durchgeführt. Als Modifizierungsmittel werden tert. organische Phosphine, die Kohlenwasserstoffreste als Substituenten haben, vorteilhaft verwendet. Bevorzugte Substituenten sind Alkylreste mit bis zu 18 Kohlenstoffatomen, Cycloalkylreste mit 5 bis 12 Kohlenstoffatomen, Aralkylreste mit 7 bis 10 Kohlenstoffatomen oder Aryl- insbesondere Phenylreste. Die Reste können gleich oder verschieden sein.

Besondere Bedeutung haben Triphenylphosphin und Alkyldiphenylphosphine erlangt. Geeignete Phosphine sind beispielsweise Triphenylphosphin, Hexyldiphenylphosphin oder substituierte Arylphosphine wie z. B. Tritolylphosphin.

Vorteilhaft wendet man die tert. Phosphinen in solchen Mengen an, daß Rhodium und Phosphor im Atomverhältnis 1 : 1 bis 1 000, insbesondere im Verhältnis 1 : 5 bis 300, vorliegen. Vorzugsweise verwendet man Rhodium in Mengen von 0,01 bis 1 Gew.-%, berechnet als Metall und bezogen auf den verwendeten 4-Pentensäureester. Es ist möglich, die Katalysatoren vor der Reaktion herzustellen. Vorteilhaft verfährt man in der Technik so, daß die Katalysatoren in situ während der Reaktion aus den einzelnen Bestandteilen, z. B. Rhodiumsalzen, Kohlenmonoxid und den angegebenen tert. organischen Phosphinen erzeugt werden.

Die erzeugten δ-Formylvaleriansäureester lassen sich aus dem Reaktionsgemisch, z. B. durch Destillation, isolieren.

Die nach dem Verfahren der Erfindung erhältlichen δ-Formylvaleriansäureester eignen sich zur Herstellung von Caprolactam, von Hexandiol oder Adipinsäure.

Das Verfahren nach der Erfindung sei in folgendem Beispiel veranschaulicht.

## Beispiel

In einem Glaskolben werden 100 g eines Gemisches aus 70 Gew.-% 3-trans- und 30 Gew.-% 3-cis Pentensäuremethylester, wie es bei der Carbonylierung von Butadien-1,3 erhalten wird, mit 10 g Katalysator vom Typ Y-Zeolith beladen mit 0,5 % Palladium bei 135 °C gerührt. Nach 6 Min. Reaktionszeit erhält man ein Reaktionsgemisch, das sich aus 8 Gew.-% 4-, 0,1 Gew.-% 2-cis-, 64,3 Gew.-% 3-trans-, 27,4 Gew.-% 3-cis- und 0,2 Gew.-% 2-trans-Pentensäureester zusammensetzt. Die Katalysatorproduktivität für 4-Pentensäuremethylester beträgt 26,6 g pro g Pd und Minute Reaktionszeit.

Nach Abtrennen des Katalysators wird das Reaktionsgemisch in einer Kolonne (Trennstufenzahl 100, Rücklauf 50) aufdestilliert. Dabei werden bei einer Kopftemperatur von 126 °C 8,2 g 4-Pentensäuremethylester mit einer Reinheit von 95 % abgenommen. Der Destillationssumpf (91,8 g) wird nach Ergänzen von 8,2 g 3-Pentenestergemisches der oben genannten Zusammensetzung erneut zur Isomerisierung eingesetzt.

180 g des nach diesem Verfahren erhaltenen Pentensäuremethylestergemisches, das 171 g (1,5 Mol) 4-Pentensäuremethylester enthält, werden in 300 g Toluol als Lösungsmittel in einem 1 1-Hubrührautoklaven der Hydroformylierung unterworfen. Das Reaktionsgemisch enthält als Katalysator 35,1 g (134 mMol) Triphenylphosphin und 54 mg (0,52 mMol) Rhodium in Form der Komplexverbindung $HRhCOL_3$ (L = $PPh_3$). Das Reaktionsgemisch wird auf 110 °C aufgeheizt und dann mit einem Gemisch aus 80 Vol.-% $H_2$ und 20 Vol-% CO ein Druck von 8 bar eingestellt. Wenn während der Reaktion der Druck im Reaktor unter 7 bar abfällt wird er durch Nachpressen eines äquimolaren Gemisches aus $H_2$ und CO wieder auf 8 bar erhöht. Nach einer Reaktionszeit von 2 Stdn. ergibt die Analyse des Reaktionsgemisches folgende Umwandlung des 4-Pentensäuremethylesters (in Mol %) :

| | |
|---|---|
| Unumgesetzter 4-PSE | 8,0 Mol-% |
| 3- und 2- PSE | 10,5 Mol-% |
| Valeriasäuremethylester | 2,6 Mol-% |
| δ-Formylvalerianester | 71,8 Mol-% |
| γ-Formylvalerianester | 6,2 Mol-% |
| Hydroxycapronsäureester | 0,5 Mol-% |
| Andere Nebenprodukte | 0,4 Mol-% |

Das Verhältnis δ-Formyl- zu γ-Formylvaleriansäureester beträgt 92 : 8. Die Ausbeute an dem eigentlichen Wertprodukt δ-Formylvaleriansäureester beträgt 78 %.

Da aber sowohl der nicht umgesetzte 4-Pentenester als auch die gebildeten 3- und 2-Isomeren nach erneuter Umwandlung in 4-Pentenester wieder für die Hydroformylierung eingesetzt werden können, ist für die Wirtschaftlichkeit entscheidend die Selektivität, das ist die Molmenge erzeugten δ-Formylvalerianesters pro Mol verbrauchten Patentesters, und die beträgt 88 %.

**Patentansprüche**

1. Verfahren zur Herstellung von δ-Formylvaleriansäureestern durch Umsetzen von Pentensäureestern mit Kohlenmonoxid und Wasserstoff unter erhöhtem Druck und bei erhöhter Temperatur in Gegenwart von Carbonylkomplexen von Metallen der 8. Gruppe des periodischen Systems, dadurch gekennzeichnet, daß man

a) 3-Pentensäureester bei erhöhter Temperatur mit Isomerisierungs-Katalysatoren behandelt und 4-Pentensäureester aus dem Reaktionsgemisch abdestilliert und

b) den so erhaltenen 4-Pentensäureester mit Kohlenmonooxid und Wasserstoff bei einer Temperatur von 80 bis 160 °C unter einem Druck von 1 bis 30 bar in Gegenwart von Carbonylkomplexen des Rhodiums, die mit tertiären organischen Phosphinen oder Phosphiten modifiziert sind, umsetzt.

2. Verfahren nach Anspruch 1, dadurch gekennzeichnet, daß als Isomerisierungskatalysatoren saure Ionenaustauscher oder saure Zeolithe mit einen Gehalt an Palladium, Rhodium oder Ruthenium verwendet.

3. Verfahren nach den Ansprüchen 1 und 2, dadurch gekennzeichnet, daß die sauren Ionenaustauscher oder sauren Zeolithe einen Gehalt an Palladium, Ruthenium oder Rhodium von 0,01 bis 1 Gew.-% haben.

4. Verfahren nach den Ansprüchen 1 bis 3, dadurch gekennzeichnet, daß man 3-Pentensäureester bei einer Temperatur von 80 bis 180 °C mit Isomerisierungskatalysatoren behandelt.

5. Verfahren nach den Ansprüchen 1 bis 4, dadurch gekennzeichnet, daß man bei der Isomerisierungs von 3-Pentensäureestern eine Verweilzeit von 0,05 bis 1 Stunde einhält.

6. Verfahren nach den Ansprüchen 1 bis 5, dadurch gekennzeichnet, daß man 4-Pentensäureester mit Kohlenmonoxid und Wasserstoff in Gegenwart von Rhodium-Carbonyl-Komplexen, die mit Triphenylphosphin modifiziert sind, umsetzt.

## Claims

1. A process for the preparation of δ-formylvalerate by reacting a pentenoate with carbon monoxide and hydrogen under superatmospheric pressure and at elevated temperature in the presence of a carbonyl complex of a metal of group VIII of the periodic table, wherein

a) a 3-pentenoate is treated with an isomerization catalyst at elevated temperature and a 4-pentenoate is distilled off from the reaction mixture, and

b) the resulting 4-pentenoate is reacted with carbon monoxide and hydrogen at from 80 to 160 °C and under from 1 to 30 bar in the presence of a rhodium carbonyl complex which has been modified with tertiary organic phosphines or phosphites.

2. A process as claimed in claim 1, wherein an acidic ion exchanger or acidic zeolite containing palladium, rhodium or ruthenium is used as the isomerization catalyst.

3. A process as claimed in claims 1 and 2, wherein the acidic ion exchanger or acidic zeolite contains from 0.01 to 1 % by weight of palladium, ruthenium or rhodium.

4. A process as claimed in claims 1 to 3, wherein a 3-pentenoate is treated with an isomerization catalyst at from 80 to 180 °C.

5. A process as claimed in claims 1 to 4, wherein the residence time in the isomerization of the 3-pentenoate is from 0.05 to 1 hour.

6. A process as claimed in claims 1 to 5, wherein a 4-pentenoate is reacted with carbon monoxide and hydrogen in the presence of a rhodium carbonyl complex which has been modified with triphenylphosphine.

## Revendications

1. Procédé de préparation d'esters d'acide δ-formylvalérianique par réaction d'esters d'acide penténique avec de l'oxyde de carbone et de l'hydrogène, sous pression élevée et à température élevée, en présence de complexes carbonyles de métaux du 8e groupe du tableau périodique, caractérisé par le fait que

a) on traite l'ester d'acide 3-penténique, à température élevée, avec des catalyseurs d'isomérisation et l'on fait distiller l'ester d'acide 4-penténique du mélange de réaction, et

b) on fait réagir l'ester d'acide 4-penténique ainsi obtenu avec de l'oxyde de carbone et de l'hydrogène à une température de 80 à 160 °C, sous une pression de 1 à 30 bar, en présence de complexes carbonyles du rhodium, qui sont modifiés avec des phosphines ou phosphites organiques tertiaires.

2. Procédé selon la revendication 1, caractérisé par le fait que, comme catalyseurs d'isomérisation, on utilise des échangeurs d'ions acides ou des zéolithes acides à teneur en palladium, rhodium ou ruthénium.

3. Procédé selon les revendications 1 ou 2, caractérisé par le fait que les échangeurs d'ions acides ou les zéolithes acides ont une teneur en palladium, rhodium ou ruthénium de 0,01 à 1 % en poids.

4. Procédé selon les revendications 1 à 3, caractérisé par le fait qu'on traite l'ester d'acide 3-penténique avec des catalyseurs d'isomérisation à une température de 80 à 180 °C.

5. Procédé selon les revendications 1 à 4, caractérisé par le fait que lors de l'isomérisation des esters d'acide 2-penténique, on maintient une durée de séjour de 0,05 à 1 heure.

6. Procédé selon les revendications 1 à 5, caractérisé par le fait que l'on fait réagir l'ester d'acide 4-penténique avec l'oxyde de carbone et l'hydrogène en présence de complexes rhodium-carbonyle qui sont modifiés avec de la triphénylphosphine.